(19) Europäisches Patentamt / European Patent Office / Office européen des brevets

(11) **EP 4 096 402 B1**

(12) **FASCICULE DE BREVET EUROPEEN**

(45) Date de publication et mention
de la délivrance du brevet:
**27.12.2023 Bulletin 2023/52**

(21) Numéro de dépôt: **22719947.8**

(22) Date de dépôt: **19.04.2022**

(51) Classification Internationale des Brevets (IPC):
*A01N 31/02* (2006.01)   *A01P 1/00* (2006.01)
*A61K 8/04* (2006.01)   *A61K 8/34* (2006.01)
*A61K 8/81* (2006.01)   *A61Q 17/00* (2006.01)

(52) Classification Coopérative des Brevets (CPC):
(C-Sets disponibles)
**A01P 1/00; A01N 31/02; A61K 8/042; A61K 8/34;
A61K 8/8147; A61Q 17/005;** A61K 2800/48  (Cont.)

(86) Numéro de dépôt international:
**PCT/EP2022/060303**

(87) Numéro de publication internationale:
**WO 2022/223553 (27.10.2022 Gazette 2022/43)**

(54) **UTILISATION DANS UNE COMPOSITION HYDROALCOOLIQUE DE POLYMERES OBTENUS PAR POLYMERISATION EN EMULSION INVERSE BASSE CONCENTRATION AVEC UN FAIBLE TAUX DE MONOMERES NEUTRALISES**

VERWENDUNG IN EINER WÄSSRIG-ALKOHOLISCHEN ZUSAMMENSETZUNG VON POLYMEREN ERHALTEN DURCH NIEDRIG KONZENTRIERTE INVERTE EMULSIONSPOLYMERISIERUNG MIT EINEM NIEDRIGEN ANTEIL AN NEUTRALISIERTEN MONOMEREN

USE IN AN HYDROALCOHOLIC COMPOSITION OF POLYMERS OBTAINED BY LOW CONCENTRATION INVERT EMULSION POLYMERISATION WITH A LOW RATIO OF NEUTRALISED MONOMERS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **22.04.2021  FR 2104192**

(43) Date de publication de la demande:
**07.12.2022  Bulletin 2022/49**

(73) Titulaire: **SNF Group
42160 Andrézieux-Bouthéon (FR)**

(72) Inventeurs:
• **BLONDEL, Frédéric
42160 ANDREZIEUX BOUTHEON (FR)**
• **BOURSIER, Thomas
42160 ANDREZIEUX BOUTHEON (FR)**

• **BOTTE, Charlène
42160 ANDREZIEUX BOUTHEON (FR)**

(74) Mandataire: **Cabinet Laurent & Charras
Le Contemporain
50 Chemin de la Bruyère
69574 Dardilly Cedex (FR)**

(56) Documents cités:
**WO-A1-2018/108611      WO-A1-2022/049354
FR-A1- 3 011 464**

**(Cont. page suivante)**

(52) Classification Coopérative des Brevets (CPC):
(Cont.)

C-Sets
**A01N 31/02, A01N 25/04**

## Description

La présente invention concerne le domaine des compositions hydroalcooliques. Plus précisément, l'invention concerne l'utilisation, pour la fabrication d'une composition hydroalcoolique, d'un copolymère obtenu par polymérisation en émulsion inverse basse concentration avec un faible taux de monomères neutralises.

## ETAT ANTERIEUR DE LA TECHNIQUE

Les virus sont à l'origine de certaines des maladies humaines les plus courantes, notamment le rhume, la varicelle et l'herpès. Certaines maladies graves, notamment celles qui sont responsables de nombreuses épidémies de la société moderne, telles que le virus Ebola, le sida, le SRAS et le SRAS-CoV-2, sont également causées par des virus.

Les virus sont des agents infectieux constitués d'un acide nucléique (ADN/ARN) dans un revêtement protéique qui s'attache aux cellules hôtes des organismes vivants et se réplique en forçant la cellule hôte à copier le matériel génétique du virus. Comme les virus peuvent se répliquer dans l'organisme malgré les mécanismes de défense de l'hôte, ils peuvent provoquer des infections chroniques à vie. En plus de leur capacité dévastatrice à provoquer des maladies graves, les virus sont également facilement transmissibles, pouvant passer par les fluides corporels, les aliments/boissons contaminés, les insectes et les contacts physiques.

Les compositions à forte teneur en alcool aident généralement à prévenir la transmission de virus et d'autres agents pathogènes par la peau car elles offrent une efficacité virucide sans irritation de la peau. Ces compositions hydroalcooliques sont des solutions aseptisantes cutanées et tuent un large éventail de virus et d'autres agents pathogènes pouvant être présents sur la peau, en particulier sur les mains. Elles agissent par contact direct et mécanique (en friction) et s'utilisent sans eau.

Des polymères épaississants sont couramment ajoutés aux compositions hydroalcooliques afin de leur donner des propriétés rhéologiques et donc de rendre leur application efficace et pratique.

Différents types de polymères formés à partir d'au moins un monomère contenant une fonction acide faible sont généralement utilisés en tant qu'agent épaississant et/ou stabilisant dans différents types d'application. On peut, par exemple, citer les brevets FR 2 810 545 et FR 2 873 126 ou encore le brevet US 3,724,547.

Le brevet EP 1 047 716 décrit des polymères à base d'acide acrylique et d'acide 2-acrylamido-2-méthylpropane sulfonique obtenus par polymérisation en émulsion inverse. Bien que ces polymères semblent avoir un effet épaississant en milieu aqueux, ils sont peu efficaces dans les compositions hydroalcooliques.

Le brevet JPH 09157130 décrit des polymères à base d'acide acrylique et d'acide 2-acrylamido-2-méthylpropane sulfonique obtenus par différentes techniques de polymérisation. Les polymères exemplifiés sont obtenus par une polymérisation par dispersion.

Toutefois les compositions hydroalcooliques connues aujourd'hui ne sont pas satisfaisantes. En effet, lors de leur utilisation, les compositions hydroalcooliques ont tendance à avoir une sensation de collant pendant l'application.

Le problème que cherche à résoudre la demanderesse est d'obtenir des compositions hydroalcooliques présentant peu de sensation de collant lors de l'application et une sensation de douceur plus importante, tout en présentant une viscosité satisfaisante.

## EXPOSE DE L'INVENTION

L'objet de cette invention est donc de proposer l'utilisation, pour la fabrication d'une composition hydroalcoolique comprenant 50% à 80% en masse d'au moins un alcool et au moins 10% en masse d'une phase aqueuse, d'un polymère branché ou réticulé composé de la répétition d'une ou plusieurs unités monomériques, dans lequel au moins une des unités monomériques comprend un groupe acrylique et au moins 30% molaire des unités monomériques sont porteuses d'au moins une fonction acide faible éventuellement sous forme neutralisée. Ledit polymère est obtenu :
par polymérisation d'une solution aqueuse d'un ou plusieurs monomères en émulsion inverse eau dans huile, dans laquelle au moins un des monomères utilisés est un monomère acrylique et au moins un des monomères utilisés est un monomère porteur d'au moins une fonction acide faible, le pourcentage molaire de monomères porteurs d'au moins une fonction acide faible par rapport à l'ensemble des monomères utilisés étant au moins de 30%, la phase aqueuse de la solution aqueuse contenant au moins un monomère jouant le rôle d'agent de ramification, de sorte que la polymérisation conduit à un polymère branché ou réticulé, et dans lequel :

i) la polymérisation est réalisée avec une concentration de la totalité des monomères en solution aqueuse appartenant à la gamme allant de 1,3 mmol à 3,6 mmol par gramme de solution aqueuse,
ii) pendant la polymérisation, au plus 20% molaire des fonctions acide présentes sur les monomères possédant au moins une fonction acide se trouvent sous forme neutralisée.

La composition hydroalcoolique ainsi fabriquée comprend 50% à 80% en masse dudit au moins un alcool, au moins 10% en masse de ladite phase aqueuse, et le polymère branché ou réticulé tel que décrit précédemment, les pourcentages étant donnés par rapport à la masse totale de la composition.

L'expression « pendant la polymérisation » signifie que, au démarrage de la polymérisation, au plus 20% molaire des fonctions acide présentes sur les monomères possédant au moins une fonction acide se trouvent sous forme neutralisée. Autrement dit, la neutralisation est réalisée préalablement à la polymérisation.

L'expression « avec une concentration de la totalité des monomères en solution aqueuse » signifie que la somme des concentrations en masse de l'ensemble des monomères en solution aqueuse appartient à la gamme allant de 1,3 mmol à 3,6 mmol par gramme de solution aqueuse. La concentration totale en monomères inclut les monomères destinés à former les unités monomériques du polymère branché ou réticulé, dits monomères « utilisés », ainsi que les monomères jouant le rôle d'agent de ramification.

La polymérisation est éventuellement suivie d'une ou plusieurs des étapes suivantes :

- une dilution ou une concentration de l'émulsion obtenue,
- une isolation pour obtenir le polymère sous la forme d'une poudre,
- une neutralisation au moins partielle des fonctions acide libre présentes dans le polymère obtenu. De préférence, la neutralisation est réalisée de sorte que le polymère comprend un pourcentage de fonctions acide neutralisées de 30% à 100% molaire par rapport à la totalité des fonctions acide présentes sur le polymère.

Un tel polymère défini par son procédé d'obtention est nommé dans la suite de la description « polymère épaississant », « polymère acrylique » ou « polymère branché ou réticulé ». L'invention a également pour objet l'utilisation d'un tel polymère épaississant, pour épaissir une composition hydroalcoolique afin d'obtenir une composition hydroalcoolique épaissie comprenant 50% à 80% en masse d'au moins un alcool et au moins 10% en masse d'une phase aqueuse. La composition épaissie comprend ainsi 50% à 80% en masse dudit au moins un alcool, au moins 10% en masse de ladite phase aqueuse, et ledit polymère épaississant.

L'invention concerne également une composition hydroalcoolique, comprenant 50% à 80% en masse d'au moins un alcool, au moins 10% en masse d'une phase aqueuse, et un polymère branché ou réticulé composé de la répétition d'une ou plusieurs unités monomériques, dans lequel au moins une des unités monomériques comprenant un groupe acrylique et au moins 30% molaire des unités monomériques sont porteuses d'au moins une fonction acide faible au moins partiellement sous forme neutralisée.

Ledit polymère est obtenu :

par polymérisation d'une solution aqueuse d'un ou plusieurs monomères en émulsion inverse eau dans huile, dans laquelle au moins un des monomères utilisés est un monomère acrylique et au moins un des monomères utilisés est un monomère porteur d'au moins une fonction acide faible, le pourcentage molaire de monomères porteurs d'au moins une fonction acide faible par rapport à l'ensemble des monomères utilisés étant au moins de 30%, la phase aqueuse de la solution aqueuse contenant au moins un monomère jouant le rôle d'agent de ramification, de sorte que la polymérisation conduit à un polymère branché ou réticulé, et dans lequel :

i) la polymérisation est réalisée avec un concentration de la totalité des monomères en solution aqueuse appartenant à la gamme allant de 1,3 mmol à 3,6 mmol par gramme de solution aqueuse,
ii) pendant la polymérisation, au plus 20% molaire des fonctions acide présentes sur les monomères possédant au moins une fonction acide se trouvent sous forme neutralisée.

Les utilisations et compositions hydroalcooliques selon l'invention présentent, de préférence, l'une ou l'autre des caractéristiques ci-après, ou une combinaison quelconque de ces caractéristiques, voire toutes les caractéristiques ci-dessous lorsqu'elles ne s'excluent pas l'une de l'autre :

- pendant la polymérisation, au plus 10%, de préférence au plus 5%, et préférentiellement au plus 2%, des fonctions acide présentes sur les monomères possédant au moins une fonction acide se trouvent sous forme neutralisée ;
- selon, un mode de réalisation particulier, toutes les fonctions acide présentes sur les monomères, soit 100% des fonctions acide, se trouvent sous forme acide libre, pendant la polymérisation ; les pourcentages indiqués sont des pourcentages molaires ;
- la polymérisation est réalisée avec une concentration de la totalité des monomères en solution aqueuse appartenant à la gamme allant de 1,7 à 3,3 mmol par gramme de solution aqueuse ;
- le polymère comporte un pourcentage molaire d'unités monomériques porteuses d'une ou plusieurs fonction(s) acide faible, par rapport à l'ensemble des unités monomériques, d'au moins 50 %, préférentiellement d'au moins 70%, très préférentiellement d'au moins 80% ;
- tous les monomères utilisés pour la préparation du polymère sont des monomères possédant au moins une insaturation éthylénique ;
- la ou les unité(s) monomérique(s) porteuse(s) d'au moins une fonction acide faible, sous forme libre, est(sont) choisie(s) parmi l'acide acrylique, l'acide méthacrylique, l'acide itaconique, l'acide crotonique, l'acide maléique et l'acide fumarique, l'acide acrylique étant préféré ;
- le polymère est un copolymère comportant au moins une unité monomérique neutre choisie parmi l'acrylamide, le méthacrylamide, le N,N-diméthylacrylamide, le N-vinylméthylacétamide, le N-vinylformamide, l'acétate de vinyle, le diacétoneacrylamide, le N-isopropyl acrylamide, le N-[2-hydroxy-1,1-bis(hydroxyméthyl) éthyl] propénamide, l'acrylate de (2-hydroxyéthyle), l'acrylate de (2,3-dihydroxypropyle), le méthacrylate de méthyle, le méthacrylate de (2-hydroxyéthyle), le méthacrylate de (2,3-dihydroxy propyle) et la vinylpyrrolidone ;
- de préférence :

  • soit toutes les unités monomériques porteuses d'au moins une fonction acide présentes dans le polymère sont des unités monomériques porteuses d'une ou plusieurs fonction(s) acide faible. En particulier, le polymère présent dans la composition est un copolymère acide acrylique/acrylamide dans lequel 30% à 100% des fonctions acide acrylique sont sous forme neutralisée ;
  • soit le polymère est un copolymère comportant au moins une unité monomérique porteuse d'une ou plusieurs fonction(s) acide fort. De manière préférée, le pourcentage molaire en unités monomériques porteuses d'une ou plusieurs fonction(s) acide fort par rapport à l'ensemble des unités monomériques est inférieur à 50%, et préférentiellement inférieur à 30%. De préférence, la ou les unité(s) monomérique(s) porteuse(s) d'une ou plusieurs fonction(s) acide fort, sous forme libre, est(sont) choisie(s) parmi les acides acrylamidoalkylsulfonique tel que l'acide 2-acrylamido-2-méthylpropane sulfonique. En particulier, le polymère présent dans la composition est un copolymère acide 2-acrylamido-2-méthylpropane sulfonique/acide acrylique ou acide 2-acrylamido-2-méthylpropane sulfonique/acide acrylique/acrylamide, dans lequel 30% à 100% des fonctions acide sont sous forme neutralisée ;

- l'agent de ramification est choisi parmi le méthylènebisacrylamide (MBA), l'éthylène glycol di-acrylate, le polyéthylène glycol diméthacrylate, le diacrylamide, le cyanométhylacrylate, le vinyloxyéthylacrylate, le vinyloxyméthacrylate, la triallylamine, le chlorure de tétraallylammonium (TAAC), le formaldéhyde, le glyoxal, les glycidyléthers comme l'éthylèneglycol diglycidyléther, les époxy, et leurs mélanges ;
- la quantité d'agent de ramification dans la composition est comprise entre 5 ppm et 10 000 ppm en masse par rapport à la masse totale de monomères, et préférentiellement entre 100 ppm et 5 000 ppm ;
- la réaction de polymérisation est réalisée en présence d'un agent émulsionnant eau dans huile ;
- la composition comprend de 0,01% à 10% en masse de polymère branché ou réticulé, par rapport à la masse totale de la composition, et préférentiellement de 0,1% à 5% en masse de polymère branché ou réticulé ;
- la composition comprend au moins un additif, et notamment au moins un auxiliaire de formulation, par exemple choisi parmi les agents chélatants, les diluants, les agents de neutralisation et d'ajustement du pH, les opacifiants, les conservateurs, les agents d'étalement, les émollients, les polymères filmogènes, les antioxydants, les parfums, les agents réfléchissants, les agents coalescents et les mélanges de ceux-ci ;
- la composition comprend de 60% à 70% en masse d'au moins un alcool par rapport à la masse totale de la composition ;
- l'alcool compris dans la composition hydroalcoolique est avantageusement choisi parmi le méthanol, l'éthanol, l'isopropanol, le butanol, le pentanol, l'hexanol, leurs isomères et leurs mélanges. Préférentiellement, l'alcool est choisi parmi l'éthanol et/ou l'isopropanol ;
- la composition hydroalcoolique comprend :

  a) 50 % à 80 % en masse dudit au moins un alcool,
  b) 0,1 % à 10 % en masse du copolymère branché ou réticulé,
  c) 10 % à 49,9 % en masse d'eau,
  d) 0 % à 10 % en masse d'au moins un additif.

- la composition hydroalcoolique comprend :

  a) 60 % à 75 % en masse d'au moins un alcool,
  b) 0,1 % à 5 % en masse du copolymère branché ou réticulé,
  c) 25 % à 39,9 % en masse d'eau,
  d) 0 % à 10% en masse d'au moins un additif.

Les polymères utilisés dans le cadre de l'invention et leur procédé d'obtention vont tout d'abord être décrits.

Les polymères utilisés dans le cadre de l'invention, sont composés de la répétition d'une ou plusieurs unités monomériques, avec au moins une des unités monomériques qui correspond à un monomère comportant un groupe acrylique. En d'autres termes, ces polymères correspondent à des homopolymères obtenus par polymérisation d'un monomère comportant un groupe acrylique ou à des copolymères obtenus par copolymérisation d'un mélange de monomères, dont un au moins comporte un groupe acrylique. Par souci de simplicité, dans la suite de la description, de tels polymères pourront simplement être nommés polymères acryliques.

De manière à remplir efficacement leur rôle d'épaississant, les polymères utilisés dans le cadre de l'invention sont hydrosolubles ou hydro-gonflants. Les monomères utilisés pour la préparation de ces polymères et en particulier le taux de monomères hydrophiles seront sélectionnés de manière à obtenir de telles propriétés.

Par polymère hydrosoluble, on entend un polymère qui, mis en solution à l'aide d'une agitation dans de l'eau à une température de 25°C à une concentration de 50g/l, donne une solution exempte de particules insolubles.

Par polymère hydro-gonflant, on entend un polymère qui, mis en solution dans de l'eau à une température de 25°C, gonfle et épaissit la solution.

Les polymères utilisés dans le cadre de l'invention sont branchés ou réticulés. Par polymères branchés, on entend, de manière classique, les polymères non linéaires qui possèdent des chaînes latérales. Les polymères branchés, incluent notamment les polymères en forme d'étoile (« star » en anglais) et en forme de peigne (« comb » en anglais). Par polymère réticulé, on entend, de manière classique, un polymère non linéaire qui se présente sous la forme d'un réseau tridimensionnel insoluble dans l'eau, mais gonflable dans l'eau.

La réticulation est obtenue en mettant en oeuvre un agent de ramification lors de la polymérisation qui est intégré dans la phase aqueuse. Un tel agent de ramification correspond à un monomère comportant deux ou plus insaturations éthyléniques. Il est, par exemple, choisi parmi le méthylènebisacrylamide (MBA), l'éthylène glycol di-acrylate, le polyéthylène glycol diméthacrylate, le diacryamide, le cyanométhylacrylate, le vinyloxyéthylacrylate, le vinyloxyméthacrylate, la triallylamine, le chlorure de tétraallylammonium (TAAC), le formaldéhyde, le glyoxal, les glycidyléthers comme l'éthylèneglycol diglycidyléther, les époxy et leurs mélanges.

Il convient de préciser que, dans le cadre de l'invention, la concentration totale en monomères donnée en relation avec le procédé de polymérisation inclut les monomères jouant le rôle d'agent de ramification.

Dans le cadre de l'invention, la demanderesse s'est intéressée à l'utilisation de polymères acryliques correspondants ou obtenus à partir d'une émulsion inverse de polymères préparée par polymérisation en émulsion inverse eau dans huile avec utilisation d'un pourcentage molaire élevé de monomères porteurs d'une ou plusieurs fonction(s) acide faible par rapport à l'ensemble des monomères utilisés, et en particulier comportant au moins 30 % molaire de monomères porteurs d'au moins une fonction acide faible. Avec un tel taux de monomères porteurs d'une fonction acide faible, les inventeurs ont mis en évidence que les propriétés du polymère obtenu sont réellement dépendantes, d'une part, du taux de neutralisation des fonctions acides des monomères utilisés lors de la polymérisation et, d'autre part, de la concentration totale des monomères dans la phase aqueuse. De manière originale par rapport aux approches proposées dans l'art antérieur qui préconisent de mener la polymérisation avec un taux important de neutralisation des fonctions acide, la demanderesse s'est orientée, dans le cadre de l'invention, vers un procédé de polymérisation en émulsion inverse de polymères présentant un faible taux de neutralisation et, notamment, un taux de neutralisation des fonctions acide d'au plus 20% molaire.

Dans le cadre de l'invention, la demanderesse propose d'utiliser un tel polymère, obtenu par polymérisation d'une solution aqueuse de monomères en émulsion inverse eau dans huile, dans lequel la polymérisation est réalisée avec une concentration de la totalité des monomères appartenant à la gamme allant de 1,3 mmol à 3,6 mmol par gramme de solution aqueuse. De plus, la demanderesse a démontré qu'une telle gamme de concentrations, contrairement aux concentrations plus importantes notamment utilisées dans l'art antérieur, était compatible avec l'obtention de polymère avec un faible taux de neutralisation des fonctions acide faible présentes et permettait de s'affranchir des problèmes de stabilité constatés dans l'art antérieur.

Dans le cadre de l'invention, le polymère utilisé est obtenu en mettant en oeuvre un procédé de préparation d'un polymère par polymérisation d'une solution aqueuse d'un ou plusieurs monomères en émulsion inverse eau dans huile, dans lequel un ou plusieurs des monomères utilisés comportent au moins une fonction acide, le pourcentage molaire de monomères porteurs d'au moins une fonction acide faible par rapport à l'ensemble des monomères utilisés étant au moins de 30%, et dans lequel :

i) la polymérisation est réalisée avec une concentration de la totalité des monomères en solution aqueuse appartenant à la gamme allant de 1,3 mmol à 3,6 mmol par gramme de solution aqueuse,
ii) lors de la polymérisation, au plus 20% molaire des fonctions acide présentes sur les monomères utilisés possédant au moins une fonction acide se trouvent sous forme neutralisée.

En particulier, lors de la polymérisation, au plus 10%, de préférence au plus 5%, et préférentiellement au plus 2%, des fonctions acides présentes sur les monomères utilisés possédant au moins une fonction acide se trouvent sous forme neutralisée, ce qui permet d'obtenir des propriétés épaississantes encore plus avantageuses. Selon un mode de réalisation particulier, 100% des fonctions acide présentes sur les monomères utilisés se trouvent sous forme acide libre, lors de la polymérisation.

Dans le cadre de l'invention, de manière optimale, la polymérisation est réalisée avec une concentration totale en monomères présents dans la solution aqueuse appartenant à la gamme allant de 1,7 à 3,3 mmol par gramme de solution aqueuse. Dans le cadre de l'invention, les concentrations en monomères sont données par rapport à la masse totale de solution aqueuse (également nommée phase aqueuse), c'est à dire masse de monomères comprise.

Notamment, il est donc possible de mener la polymérisation avec les combinaisons suivantes :

- Une concentration de la totalité des monomères en solution aqueuse appartenant à la gamme allant de 1,3 mmol à 3,6 mmol par gramme de solution aqueuse, avec au plus 20% molaire, avantageusement au plus 10%, de préférence au plus 5%, et préférentiellement au plus 2%, voire 0% des fonctions acide présentes sur les monomères possédant au moins une fonction acide qui se trouvent sous forme neutralisée,
- Une concentration de la totalité des monomères en solution aqueuse appartenant à la gamme allant de 1,7 mmol à 3,3 mmol par gramme de solution aqueuse, avec au plus 20% molaire, avantageusement au plus 10%, de préférence au plus 5%, et préférentiellement au plus 2%, voire 0% des fonctions acide présentes sur les monomères possédant au moins une fonction acide qui se trouvent sous forme neutralisée.

Le pourcentage molaire en monomères porteurs d'au moins une fonction acide faible par rapport à l'ensemble des monomères utilisés est préférentiellement au moins de 50%, préférentiellement d'au moins 70%, très préférentiellement d'au moins 80%. De tels pourcentages molaires peuvent être utilisés, avec n'importe laquelle des combinaisons concentration en monomères/taux de neutralisation précédemment citées.

Dans le cadre de l'invention, on réalisera, de préférence, la polymérisation avec des monomères qui possèdent tous au moins une insaturation éthylénique.

De manière préférée, la polymérisation est réalisée avec un seul monomère porteur d'au moins une fonction acide faible dont le pourcentage molaire par rapport à l'ensemble des monomères utilisés est au moins de 30%, qui sous forme libre est choisi parmi l'acide acrylique, l'acide méthacrylique, l'acide itaconique, l'acide crotonique, l'acide maléique et l'acide fumarique. Le monomère porteur d'au moins une fonction acide faible est très préférentiellement l'acide acrylique sous forme libre ou avec un taux de neutralisation conforme à l'invention. Il est également possible d'utiliser plusieurs monomères porteurs d'au moins une fonction acide faible, notamment choisis parmi ceux précédemment listés, dont le pourcentage molaire total par rapport à l'ensemble des monomères utilisés est au moins de 30%. De préférence, l'un de ces monomères est l'acide acrylique sous forme libre ou avec un taux de neutralisation conforme à l'invention.

La polymérisation peut être réalisée avec au moins un monomère porteur d'au moins une fonction acide fort. Dans ce cas, la polymérisation est, de préférence, réalisée avec une concentration en monomères porteurs d'au moins une fonction acide fort par rapport à l'ensemble des monomères utilisés de moins de 50%, et préférentiellement de moins de 30%. La polymérisation peut, par exemple, être menée avec un monomère porteur d'au moins une fonction acide fort qui sous forme libre est choisi parmi les acides acrylamidoalkylsulfoniques, tel que l'acide 2-acrylamido-2-méthylpropane sulfonique (ATBS). Dans ce cas, la polymérisation peut, par exemple, être menée avec une combinaison acide acrylique/ATBS ou acide acrylique/ATBS/acrylamide, les monomères acides pouvant se trouver sous forme libre ou avec un taux de neutralisation conforme à l'invention.

Dans le cadre de l'invention, il a été constaté qu'en sélectionnant une concentration en monomères appartenant à la gamme allant de 1,3 mmol à 3,6 mmol par gramme de solution aqueuse pour réaliser la réaction de polymérisation en émulsion inverse, il était possible de préparer des émulsions inverses de polymères porteurs de fonction acide avec un faible taux de neutralisation, voire aucune neutralisation, qui soient stables, c'est-à-dire sans l'observation d'un phénomène rapide de précipitation. De surcroît, il a été démontré qu'une telle gamme de concentrations, contrairement aux concentrations plus importantes notamment utilisées dans l'art antérieur, associée à une faible neutralisation des fonctions acides présentes, permettait d'obtenir des polymères procurant de meilleures propriétés sensorielles à l'application, après une étape de neutralisation au moins partielle, supérieure aux polymères de l'art antérieur obtenus par polymérisation en émulsion inverse.

Par « monomère porteur d'au moins une fonction acide », on entend un monomère porteur d'une ou plusieurs fonction(s) acide libre ou neutralisée (c'est-à-dire salifiée par action d'une base). Le terme « fonction acide », sans plus de précision désigne donc à la fois les fonctions acides sous forme libre et sous forme neutralisée. Lorsqu'un monomère comporte plus d'une fonction acide, il est possible de n'avoir qu'une partie des fonctions acide sous forme neutralisée. La ou les fonctions acides présente(s) peu(ven)t être une fonction acide faible ou acide fort. En général, les monomères utilisés ne comporteront que des fonctions acides faible ou que des fonctions acides fort, et le plus souvent, des monomères porteurs d'une seule fonction acide seront utilisés. Les mêmes définitions et préférences s'appliquent aux unités monomériques présentes sur le polymère obtenu.

A titre d'exemple de monomère porteur d'au moins une fonction acide faible sous forme libre, du type -COOH, on peut citer l'acide acrylique, l'acide méthacrylique, l'acide itaconique, l'acide crotonique, qui comportent tous une seule fonction acide faible, et l'acide maléique et l'acide fumarique qui comportent, quant à eux, deux fonctions acide faible.

A titre d'exemple de monomère porteur d'une fonction acide fort sous forme libre, on peut citer les monomères porteurs d'une fonction acide phosphonique ou acide sulfonique tels que les acides acrylamidoalkylsulfoniques tel que l'acide 2-acrylamido-2-méthylpropane sulfonique.

Sous leur forme neutralisée, les fonctions acides sont sous forme anionique avec un contre-ion ou cation dépendant de la base utilisée pour la neutralisation, par exemple du type $Na^+$ quand la soude est utilisée ou encore $NH_4^+$ quand l'ammoniaque est utilisée. De manière classique, le contrôle du nombre de fonctions acide sous forme neutralisée est assuré par le choix du pH de la solution aqueuse de monomères qui sera ajusté en fonction du pKa des fonctions acides présentes.

La polymérisation peut mettre en jeu un seul type de monomère, alors choisi parmi les monomères porteurs d'au moins une fonction acide faible ou différents types de monomères dont un au moins est porteur d'au moins une fonction acide faible, avec une proportion des fonctions acide présentes sur les monomères utilisés, et donc sur le copolymère obtenu, sous une forme neutralisée qui est inférieure ou égale à 20% molaire. En particulier, en plus du ou des unités monomériques porteuses d'au moins une fonction acide faible précédemment décrits, le polymère obtenu peut contenir d'autres unités monomériques telles que des unités monomériques porteuses d'au moins une fonction acide fort, des unités monomériques neutres (ou non-ioniques), des unités monomériques cationiques et/ou encore des unités monomériques à caractère hydrophobes. Quel que soit le cas, les conditions de formation de la phase aqueuse et de polymérisation sont telles que les fonctions acide des monomères mis en jeu restent majoritairement sous forme libre, et ne soient pas neutralisées par formation d'une forme salifiée, ou faiblement neutralisées avec un taux de neutralisation limité inférieur ou égal à 20% molaire. Lorsqu'une neutralisation inférieure ou égale à 20% molaire a lieu, elle est en général menée dans la phase aqueuse, par ajout d'une quantité de base appropriée. Une base telle que la soude ou l'ammoniaque pourra être utilisée.

Notamment, la réaction de polymérisation peut être réalisée avec au moins un monomère neutre choisi parmi l'acrylamide, le méthacrylamide, le N,N-diméthylacrylamide, le N-vinylméthylacétamide, le N-vinylformamide, l'acétate de vinyle, le diacétoneacrylamide, le N-isopropyl acrylamide, le N-[2-hydroxy-1,1-bis(hydroxyméthyl) éthyl] propénamide, l'acrylate de (2-hydroxyéthyle), l'acrylate de (2,3-dihydroxypropyle), le méthacrylate de méthyle, le méthacrylate de (2-hydroxyéthyle), le méthacrylate de (2,3-dihydroxy propyle), la vinylpyrrolidone, ou d'autres esters acryliques, ou d'autres esters à insaturation éthylénique. Par exemple, la polymérisation peut être réalisée avec de 30 à 99% molaire d'au moins un monomère possédant une ou plusieurs fonction(s) acide faible et de 1 à 70% molaire d'au moins un monomère neutre. La polymérisation peut, par exemple, être menée avec une combinaison acide acrylique/acrylamide, l'acide acrylique étant sous forme neutre ou avec un taux de neutralisation conforme à l'invention.

Il est également possible de réaliser une copolymérisation avec au moins un monomère cationique. A titre d'exemple de monomères cationiques, on peut citer les sels de diallyldialkyl ammonium comme le chlorure de diallyl diméthyl ammonium (DADMAC) ; les sels acidifiés ou quaternisés d'acrylates et méthacrylates de dialkylaminoalkyle, en particulier d'acrylate de dialkylaminoéthyle (ADAME) et de méthacrylate de dialkylaminoéthyle (MADAME) ; les sels acidifiés ou quaternisés de dialkyl- aminoalkylacrylamides ou méthacrylamides, comme par exemple le méthacrylamido-propyl triméthyl ammonium chlorure (MAPTAC), l'acrylamido-propyl triméthyl ammonium chlorure (APTAC) et les produits de Mannich comme les dialkylaminométhylacrylamides quaternisés.

Les sels acidifiés sont obtenus par les moyens connus de l'homme de métier, et notamment par protonation. Les sels quaternisés sont également obtenus par les moyens connus de l'homme du métier notamment, par réaction avec le chlorure de benzyle, le chlorure de méthyle (MeCl), les chlorures d'aryle, d'alkyle, ou le diméthylsulfate.

Il est également possible de réaliser une copolymérisation avec au moins un monomère à caractère hydrophobe. A titre d'exemple de monomères à caractère hydrophobes, on peut citer l'acide undécanoïque acrylamide, l'acide un-dodécyl méthyl acrylamide, les dérivés d'acide acrylique comme les alkyles acrylates ou méthacrylates comme par exemple le méthacrylate de béhényl 25 éthoxylé. Dans ce cas, le pourcentage molaire en monomères à caractère hydrophobe par rapport à l'ensemble des monomères utilisés de moins de 10%, et généralement compris entre 0.001% et 7%.

Selon une première variante du procédé selon l'invention, tous les monomères porteurs d'au moins une fonction acide utilisés pour mener la polymérisation sont des monomères porteurs d'au moins une fonction acide faible.

Selon une seconde variante du procédé selon l'invention, la polymérisation est réalisée avec au moins un monomère porteur d'au moins une fonction acide fort, en plus d'au moins un monomère porteur d'au moins une fonction acide faible. Dans ce cas, le pourcentage molaire en monomères porteurs d'au moins une fonction acide fort par rapport à l'ensemble des monomères utilisés est préférentiellement de moins de 50%, très préférentiellement de moins de 30%.

Les copolymères obtenus selon le procédé de l'invention peuvent notamment être formés d'une combinaison d'au moins une unité monomérique porteuse d'au moins une fonction acide faible et d'au moins une unité monomérique porteuse d'au moins une fonction acide fort, et notamment correspondre à un copolymère acide acrylique/ATBS, ces monomères acide étant sous forme neutre ou avec un taux de neutralisation conforme à l'invention ; d'une combinaison d'au moins une unité monomérique porteuse d'au moins une fonction acide faible avec au moins une unité monomérique neutre et éventuellement au moins une unité monomérique porteuse d'au moins une fonction acide fort, et notamment correspondre à un copolymère acide acrylique/acrylamide ou à un copolymère acide acrylique/ATBS/acrylamide, l'acide acrylique et l'ATBS étant sous forme neutre ou avec un taux de neutralisation conforme à l'invention ; d'une combinaison d'au moins une unité monomérique porteuse d'au moins une fonction acide faible avec au moins une unité monomérique cationique et éventuellement au moins une unité monomérique porteuse d'au moins une fonction acide fort ; ou encore d'une combinaison d'au moins une unité monomérique porteuse d'au moins une fonction acide faible avec au moins une unité monomérique neutre et au moins un monomère cationique et éventuellement au moins une unité monomérique porteuse d'au moins une fonction acide fort.

Dans le procédé de polymérisation en émulsion inverse utilisé dans le cadre de l'invention, les monomères sont mis en solution aqueuse. Cette solution aqueuse correspond à la phase aqueuse de l'émulsion inverse. Dans le cadre de l'invention, dans la solution aqueuse utilisée pour la polymérisation, au plus 20% molaire des fonctions acide présentes sur les monomères possédant au moins une fonction acide se trouvent sous forme neutralisée.

Il est possible d'utiliser un agent de transfert, autrement appelé agent limitateur de chaîne. L'utilisation d'un agent de transfert est particulièrement avantageuse pour contrôler le poids moléculaire du polymère obtenu. A titre d'exemple, d'agent de transfert, on peut citer le méthanol, l'isopropanol, l'hypophosphite de sodium, le 2-mercaptoéthanol, le méthallysulfonate de sodium et leur mélange. L'homme du métier ajustera, de manière connue, les quantités utilisées d'agent de ramification et éventuellement d'agent de transfert, en fonction de s'il souhaite obtenir un polymère branché ou réticulé.

De manière plus détaillée, le procédé utilisé dans le cadre de l'invention comprend les étapes suivantes :

    a) Disposer d'une solution aqueuse du ou des monomères sélectionnés, dite phase aqueuse,
    b) Emulsionner ladite solution aqueuse dans une phase non miscible à l'eau, dite phase huile,
    c) Mener la réaction de polymérisation.

Bien entendu, la solution aqueuse de l'étape a) présente une concentration totale en monomères, un pourcentage molaire de monomères porteurs d'au moins une fonction acide faible par rapport à l'ensemble des monomères utilisés et un taux de neutralisation des fonctions acide présentes sur les monomères possédant au moins une fonction acide conformes au procédé décrit dans le cadre de l'invention.

En général, la réaction de polymérisation est réalisée en présence d'un agent émulsionnant eau dans huile. Ce dernier est le plus souvent introduit dans la phase huile dans laquelle la solution aqueuse est émulsionnée. Par agent émulsifiant du type eau dans huile (E/H), on entend un agent émulsifiant possédant une valeur HLB suffisamment faible pour fournir des émulsions eau dans huile, et notamment une valeur HLB inférieure à 10.

La valeur de HLB est calculée selon la relation suivante :

$$HLB = (\% \text{ en poids de la partie hydrophile}) / 5$$

Le pourcentage en poids de la partie hydrophile étant le rapport entre le poids moléculaire de la partie hydrophile sur le poids moléculaire total de la molécule.

A titre d'exemple de tels agents émulsifiants eau dans huile, on peut citer les polymères tensioactifs tels que les polyesters de poids moléculaire compris entre 1000 et 3000, produits de la condensation entre un acide poly(isobutènyl) succinique ou son anhydride et un polyéthylène glycol, les copolymères à blocs de poids moléculaire compris entre 2500 et 3500, par exemple ceux commercialisés sous les noms HYPERMER®, les extraits de sorbitan, comme le monooléate de sorbitan, l'isostéarate de sorbitan ou le sesquioléate de sorbitan, certains esters de sorbitan polyéthoxylés, comme le monooléate de sorbitan pentaéthoxylé ou l'isostéarate de sorbitan pentaéthoxylé, ou encore l'alcool oléocétylique diéthoxylé, l'acrylate de lauryle tétraéthoxylé.

Dans le procédé de polymérisation en émulsion inverse, la solution aqueuse contient le ou les monomère(s) et éventuellement l'agent de ramification et l'agent de transfert. Elle peut également contenir des agents complexants comme l'éthylène diamine ou l'acide éthylène diamine tétraacétique.

Le plus souvent, la réaction de polymérisation de l'étape c) est amorcée par introduction dans l'émulsion formée à l'étape b) d'un initiateur de radicaux libres. A titre d'exemple d'agents initiateurs de radicaux libres, on peut citer les couples oxydant-réducteur avec parmi les oxydants l'hydroperoxyde de cumène ou le butylhydroxypéroxyde tertiaire, et parmi les réducteurs les persulfates tels que le métabisulfite de sodium et le sel de Mohr. Des composés azoïques tels que le 2,2'-azobis(isobutyronitrile) et le chlorhydrate de 2,2'-azobis(2amidinopropane) peuvent également être utilisés.

De manière classique, la polymérisation est généralement menée de manière isotherme, adiabatique ou à température contrôlée. C'est-à-dire que l'on maintient la température constante généralement entre 10 et 50°C (isotherme), ou alors on laisse la température augmenter naturellement (adiabatique) et dans ce cas on démarre généralement la réaction à une température inférieure à 10°C et la température finale est généralement supérieure à 50°C, ou enfin on contrôle la montée en température pour avoir une courbe de température entre celle de l'isotherme et celle de l'adiabatique.

Il est possible d'introduire à la fin de la réaction de polymérisation, un ou plusieurs agents émulsifiants huile dans eau, de préférence à une température inférieure à 50°C.

Par agent émulsifiant du type huile dans eau (H/E), on entend un agent émulsifiant possédant une valeur HLB suffisamment élevée pour fournir des émulsions huile dans l'eau et notamment une valeur HLB supérieure à 10. A titre d'exemple de tels agents émulsifiants huile dans eau, on peut citer les esters de sorbitan éthoxylés comme l'oléate de sorbitan éthoxylé avec 20 équivalents d'oxyde d'éthylène (EO 20, soit 20 répétitions du motif oxyde d'éthylène dans la molécule), le laurate de sorbitan polyéthoxylé avec 20 moles d'oxyde d'éthylène, l'huile de ricin polyéthoxylé avec 40 moles d'oxyde d'éthylène, l'alcool oléodécylique décaéthoxylé, l'alcool laurique heptaéthoxylé, ou le monostéarate de sorbitan polyéthoxylé avec 20 moles d'oxyde d'éthylène.

Les quantités d'agent(s) émulsifiant(s) introduites sont telles que l'émulsion inverse obtenue, contiendra généralement de 1% à 10% en masse, et de préférence de 2,5% à 9% en masse, d'agents émulsifiants du type eau dans huile (E/H) et, éventuellement, de 2% à 10% en masse, et de préférence de 2,5% à 6% en masse d'agents émulsifiants du type huile dans eau (H/E).

En général, le rapport massique de la phase aqueuse sur la phase huile est de 50/50 à 90/10.

La phase huile utilisée dans le procédé de polymérisation en émulsion inverse peut être composée, par exemple, d'une huile minérale, notamment commerciale, contenant des hydrocarbures saturés de type paraffinique, isoparaffinique, cycloparaffinique, naphtalique présentant à température ambiante (22°C), une densité entre 0,7 et 0,9 ; d'une huile végétale ; d'une huile de synthèse tel que le polydécène hydrogéné ou le polyisobutène hydrogéné ; d'un ester tel que l'octyl stéarate ou le buthyl oléate ; d'une huile végétale comme le squalane ou l'hémisqualane; ou d'un mélange de plusieurs de ces huiles.

A la fin de la réaction de polymérisation, il est également possible que l'émulsion obtenue soit diluée ou concentrée. En particulier, il est possible de concentrer par distillation, l'émulsion obtenue ou bien de complètement la sécher, afin d'obtenir une poudre. Une telle concentration ou séchage sera mené avec ou sans introduction préalable d'agent émulsifiant du type huile dans eau (H/E).

Les émulsions inverses ainsi obtenues peuvent être concentrées, par exemple par distillation. On obtient alors des émulsions inverses dont la concentration en polymère peut être comprise entre 30 et 75% en masse, préférentiellement entre 40 et 65% en masse.

Les polymères obtenus à partir des émulsions inverses soumises ultérieurement à une étape d'isolation, peuvent se trouver sous la forme d'une poudre. Une telle étape d'isolation peut, par exemple, être choisie parmi les techniques de précipitation, de distillation azéotropique et de séchage par atomisation et pulvérisation.

En effet, dans le cadre de l'invention, il est possible de concentrer ou d'isoler le polymère sous la forme d'une émulsion inverse obtenue directement en sortie du procédé de polymérisation en émulsion inverse, sans perdre les propriétés avantageuses des polymères obtenus. Il existe en particulier de nombreux procédés d'obtention de poudre à partir d'émulsions inverses de polymères qui consistent à isoler la matière active des autres constituants de l'émulsion comme par exemple :

- la précipitation dans un milieu non-solvant tel que l'acétone, le méthanol ou tout autre solvant polaire dans lequel le polymère n'est pas soluble. Une simple filtration permet alors d'isoler la particule de polymère.
- la distillation azéotropique en présence d'agent agglomérant et de polymère stabilisant permet de conduire à des agglomérats que l'on isole facilement par filtration avant de procéder au séchage de la particule.
- le "spray-drying" ou séchage par atomisation consiste à créer un nuage de fines gouttelettes d'émulsion dans un courant d'air chaud, pendant une durée contrôlée.

Les polymères obtenus après de telles étapes gardent leurs propriétés avantageuses, en termes de capacité épaississante et en termes de résistance aux électrolytes.

Sans étape de neutralisation additionnelle, dans les polymères obtenus à l'issu du procédé de polymérisation en émulsion inverse ou après une étape de séchage ou de concentration, au plus 20% molaire des fonctions acide présentes sont sous forme neutralisée, de préférence au plus 10%, de manière encore plus préférée au plus 5%, et préférentiellement au plus 2%. Ce faible taux de neutralisation des fonctions acide présentes offre au formulateur une grande souplesse, lui permettant d'ajuster les propriétés du polymère et donc l'effet épaississant souhaité, en ajustant à façon, le taux de neutralisation. Une telle approche permet également au formulateur de choisir la nature de l'agent neutralisant utilisé, compatible avec l'utilisation ciblée.

Pour obtenir les effets désirés, la polymérisation est le plus souvent suivie d'une étape de neutralisation, autrement appelée étape de post-neutralisation, d'au moins une partie, voire de la totalité, des fonctions acides libres présent sur le polymère. Dans le cas où une étape de neutralisation au moins partielle des fonctions acide libres présentes dans le polymère obtenu est menée après la réaction de polymérisation, elle conduit, de préférence, à un pourcentage de neutralisation par rapport à la totalité des fonctions acide présentes sur le polymère de 30 à 100%.

Une telle étape de post-neutralisation peut être réalisée de différentes manières :

- La post-neutralisation peut être faite sur l'émulsion inverse obtenue à l'issue du procédé de polymérisation en émulsion inverse. Généralement cela est le cas, lorsque le fabricant neutralise lui-même le polymère sous forme d'émulsion inverse.
- La post-neutralisation peut être faite sur une solution aqueuse obtenue suite à l'inversion de l'émulsion inverse dans de l'eau. Généralement, cela est le cas lorsque le formulateur met en oeuvre l'émulsion inverse, ou la poudre en découlant, dans une solution aqueuse, appelée solution mère, avant d'ajouter cette dernière dans la composition à épaissir. Il a alors la liberté d'ajuster la concentration en polymère de la solution, le taux de neutralisation et la nature des agents neutralisant.
- La post-neutralisation peut aussi être réalisée sur la composition dans laquelle l'émulsion inverse ou la poudre en découlant a été incorporée. De la même manière que le cas précédent, l'utilisateur a la liberté d'ajuster le taux de neutralisation et la nature des agents neutralisants.

La neutralisation est effectuée grâce à une base, de manière similaire à la neutralisation des monomères précédemment décrite dans la cadre du procédé de polymérisation, dont la nature et les quantités sont sélectionnées par l'homme du métier.

Ces polymères ainsi neutralisés offrent de bien meilleures propriétés épaississantes et de meilleures propriétés sensorielles, toutes conditions égales par ailleurs, comparés aux polymères obtenus par polymérisation en émulsion inverse ne satisfaisant pas les conditions de concentration et de neutralisation des monomères telles que définies dans le procédé selon l'invention. Particulièrement après neutralisation, les polymères offrent des propriétés avantageuses par rapport à des polymères constitués des mêmes monomères, mais préparés par polymérisation en émulsion inverse directement à des taux de neutralisation supérieurs et/ou à une concentration totale en monomères différente.

De manière avantageuse, les polymères utilisés dans le cadre de l'invention permettent après complète neutralisation des fonctions acide libres présentes, ou du moins neutralisation plus importante, d'épaissir bien plus efficacement des milieux aqueux présents dans des compositions hydroalcooliques.

Le procédé de préparation des compositions hydroalcooliques selon l'invention, et en particulier l'incorporation des polymères précédemment décrits, vont maintenant être détaillés.

La fabrication de compositions hydroalcooliques est largement connue par l'homme de l'art. L'ajout du polymère acrylique épaississant précédemment décrit peut se faire à n'importe quelle étape de la fabrication de la composition hydroalcoolique. Préférentiellement, dans une première étape on épaissit une solution aqueuse pouvant contenir des additifs par l'intermédiaire de l'agent épaississant. Dans une seconde étape on ajoute progressivement le ou les alcools sous agitation. Si une neutralisation est nécessaire elle s'effectue préférentiellement en fin de préparation dans le cas de polymère épaississant sous forme d'émulsion inverse.

La composition hydroalcoolique comprend de préférence de 0,01% à 10% en masse de polymère acrylique épaississant, et préférentiellement de 0,1 à 5% en masse, ces pourcentages étant donnés par rapport à la masse totale de la composition hydroalcoolique.

La composition hydroalcoolique a une viscosité comprise entre 500 et 8000 cps, de manière préférentielle comprise entre 700 et 6000 cps, plus préférentiellement comprise entre 1000 et 5500 cps. La viscosité est mesurée à 25°C avec un appareil Brookfiled de type RVT (vitesse de rotation 20 t/min), appartenant à la gamme allant de 2000 mPa.s à 100 000 mPa.s, notamment à la gamme allant de 3000 mPa.s à 50 000 mPa, s.

L'étape de neutralisation conduisant à un pourcentage de fonctions acide neutralisées de 30 à 100% par rapport à la totalité des fonctions acide présentes sur le polymère peut se faire avant ou après l'incorporation du polymère dans la composition.

Par ailleurs, le polymère obtenu par polymérisation en émulsion inverse selon le procédé précédemment décrit conserve ses propriétés avantageuses, qu'il se présente sous la forme d'une émulsion inverse plus ou moins concentrée, d'une poudre ou d'une solution aqueuse. Par conséquent, le polymère acrylique épaississant pourra être utilisé, sous la forme d'une émulsion inverse, d'une poudre ou sous forme solubilisée, par exemple dans l'eau, ou bien sous forme de dispersion aqueuse ou organique. Généralement c'est une forme solubilisée du polymère dans l'eau qui est utilisée, obtenue soit par inversion d'une émulsion inverse dans l'eau, soit par dissolution d'une poudre dans l'eau. Quelle que soit la forme sous laquelle il est introduit, au moment de son utilisation, le polymère joue son rôle d'épaississant.

De manière classique, les compositions hydroalcooliques selon l'invention peuvent comprendre au moins un additif, dont la teneur est de 0 à 10 % en masse de la composition. L'additif est avantageusement choisi parmi les tensioactifs, les agents d'ajustement du pH, les parfums, les colorants, les conservateurs, et les agents hydratants, les

EP 4 096 402 B1

agents antibactériens, les agents anticomplexants. De tels agents actifs et additifs sont bien connus du formulateur de compositions hydroalcooliques, notamment dans les domaines de la cosmétique et de la dermatologie.

Préférentiellement, la composition hydroalcoolique comprend au moins un agent hydratant. L'agent hydratant peut être choisi parmi l'allantoïne ; l'acide pyrrolidonecarboxylique et les sels de celui-ci ; l'acide hyaluronique et les sels de celui-ci ; l'acide sorbique et les sels de celui-ci ; les acides aminés, par exemple l'urée, la lysine, l'arginine, la cystéine, ou la guanidine ; les polyhydroxyalcools tels que la glycérine, le propylèneglycol, l'hexylèneglycol, l'hexanetriol, l'ethoxydiglycol, le diméthiconecopolyol et le sorbitol, ainsi que les esters de ceux-ci ; le polyéthylèneglycol ; l'acide glycolique et les sels glycolate (par exemple d'ammonium et d'alkylammonium quaternaire) ; le chitosane ; les extraits d'aloe-vera ; les extraits d'algue ; le miel et les extraits de celui-ci ; l'inositol ; l'acide lactique et les sels lactate (par exemple d'ammonium et d'alkylammonium quaternaire) ; le D-panthénol ; l'ascorbylphosphate de magnésium ; l'acide kojique ; la lactamide-monoéthandamine ; l'acétamidemonoéthanolamine ; leurs analogues, et les mélanges de ceux-ci. Préférentiellement, l'agent hydratant est la glycérine.

De manière préférentielle, la composition hydroalcoolique comprend :

a) 50 % à 80 % en masse d'au moins un alcool,
b) 0,1 % à 10 % en masse d'au moins un copolymère branché ou réticulé comme décrit cidessus,
c) 10% à 49,9 % en masse d'eau,
d) 0 % à 10% en masse d'au moins un additif.

De manière plus préférentielle la composition hydroalcoolique comprend :

a) 60 % à 75 % en masse d'au moins un alcool,
b) 0,1 % à 5 % en masse d'au moins un copolymère branché ou réticulé comme décrit cidessus,
c) 25 % à 39,9 % en masse d'eau,
d) 0 % à 10% en masse d'au moins un additif.

De manière préférentielle, la composition hydroalcoolique comprend entre 0,1% et 10% en masse d'au moins un agent hydratant, de préférence de 0,5% à 8% en masse, et plus préférentiellement, de 1% à 5% en masse, par rapport à la masse totale de la composition.

L'invention et les avantages qui en découlent ressortiront mieux des exemples suivants donnés afin d'illustrer l'invention et non de manière limitative.

## DESCRIPTION DE LA FIGURE

La figure ci-après illustre de manière non limitative des avantages et caractéristiques de l'invention :

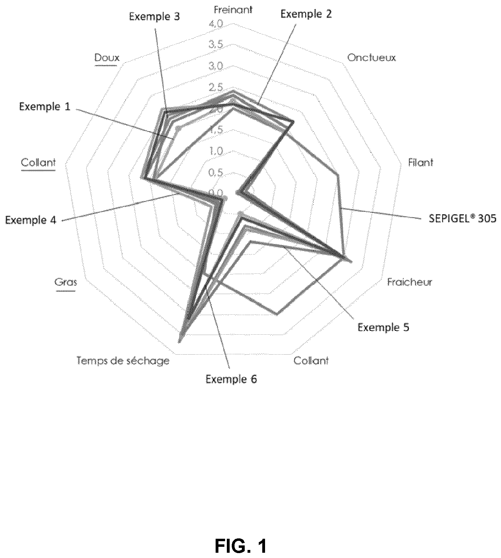

[Fig. 1] est une représentation graphique des résultats des analyses sensorielles des compositions hydroalcooliques.

## EXEMPLES DE MODES DE REALISATION DE L'INVENTION

### Exemple 1 (selon l'invention)

On charge dans un bécher de 1 L sous agitation magnétique les ingrédients de la phase aqueuse:

- 150 g d'acide acrylique glacial
- 600 g d'eau déionisée
- 0,023 g d'hypophosphite de sodium (150 ppm)
- 0,10 g de diéthylènetriaminepentacétate de sodium
- 0,075 g de méthylènebisacrylamide (500 ppm)
- 0,15 g de bromate de sodium
- 5,8 g d'hydroxyde de sodium

Ensuite dans un réacteur en verre de 1 L, sous agitation mécanique, la phase organique est préparée avec :

- 102 g d'hydrocarbure aliphatique (Isopar L)
- 98 g d'huile minérale blanche (Marcol 152)
- 20 g de monooléate de sorbitol
- 25 g de stabilisant polymérique (Hypermer 1083).

La phase aqueuse est transférée progressivement dans la phase organique. La pré-émulsion ainsi formée est alors soumise à fort cisaillement pendant 1 minute (Ultra Turrax, IKA).

L'émulsion inverse est alors dégazée pendant 30 minutes grâce à un simple barbotage d'azote.

On ajoute alors une solution aqueuse contenant 1,0 % en masse de métabisulfite de sodium à un débit de 2,5 ml/h pendant une durée de 1h30. Une fois la température maximum atteinte, on maintient la température du mélange réactionnel pendant 60 minutes avant refroidissement.

Enfin, on ajoute vers 30°C, 40 g d'alcool tridécylique éthoxylé (6 moles).

**Exemple 2 (selon l'invention)**

On charge dans un bécher de 1 L sous agitation magnétique les ingrédients de la phase aqueuse:

- 150 g d'acide acrylique glacial
- 605 g d'eau déionisée
- 0,10 g de diéthylènetriaminepentacétate de sodium
- 0,225 g de méthylènebisacrylamide (1500 ppm)
- 0,15 g de bromate de sodium

Ensuite dans un réacteur en verre de 1 L, sous agitation mécanique, la phase organique est préparée avec :

- 102 g d'hydrocarbure aliphatique (Isopar L)
- 98 g d'huile minérale blanche (Marcol 152)
- 20 g de monooléate de sorbitol
- 25 g de stabilisant polymérique (Hypermer 1083).

La phase aqueuse est transférée progressivement dans la phase organique. La pré-émulsion ainsi formée est alors soumise à fort cisaillement pendant 1 minute (Ultra Turrax, IKA).

L'émulsion inverse est alors dégazée pendant 30 minutes grâce à un simple barbotage d'azote.

On ajoute alors une solution aqueuse contenant 1,0 % en masse de métabisulfite de sodium à un débit de 2,5 ml/h pendant une durée de 1h30. Une fois la température maximum atteinte, on maintient la température du mélange réactionnel pendant 60 minutes avant refroidissement.

Enfin, on ajoute vers 30°C, 40 g d'alcool tridécylique éthoxylé (6 moles).

D'autres exemples 3 à 6, conformes à l'invention, sont préparés selon le protocole de l'exemple 1, en faisant varier le pourcentage molaire d'acide faible par rapport à l'ensemble des monomères, ainsi que le pourcentage molaire d'acide faible neutralisé.

Des contre-exemples 1 à 4, non-conformes à l'invention, sont préparés selon le protocole de l'exemple 1, en faisant varier la concentration de la totalité des monomères en solution aqueuse, le pourcentage molaire d'acide faible par rapport à l'ensemble des monomères, ainsi que le pourcentage molaire d'acide faible neutralisé.

Préparation de la composition hydroalcoolique

Dans un bécher on ajoute 82,5g d'eau déionisée puis sous agitation mécanique (tripâle - 500 tours par minute) on ajoute progressivement la quantité d'émulsion inverse (exemples 1 à 6) désirée pour obtenir une solution contenant le pourcentage en masse de polymère actif voulu. On agite pendant 5 min afin d'inverser l'émulsion. Ensuite 4,5 g de glycérine sont ajoutés à la solution. Après 1 min d'agitation l'éthanol est introduit progressivement et agité jusqu'à une parfaite homogénéisation du milieu.

La viscosité est alors mesurée à l'aide d'un viscosimètre Brookfield de type RVT avec le module 4 et vitesse de rotation 20 tours par minute.

Les polymères selon l'invention sont comparés avec un polymère épaississant disponible commercialement. Il s'agit du SEPIGEL® 305 (commercialisé par la société SEPPIC) qui est une émulsion inverse. Ce produit commercial est typiquement utilisé dans des compositions hydroalcooliques comme agent épaississant.

*Tableau 1 : composition de la composition hydroalcoolique*

| Produit | % massique |
|---------|------------|
| Eau | QSP |

(suite)

| Produit | % massique |
|---|---|
| Polymère | Voir tableaux 2 et 3 |
| Glycérine | 1,5 |
| Ethanol | 70 |

*Tableau 2 : Caractéristiques des compositions hydroalcooliques*

| | Masse phase aq (g) | [Monomères] phase aq (mmol/g) | %mol acide faible | %mol acide faible neutralisé | Processabilité | Viscosité GHA 0,5% polymère (cps) |
|---|---|---|---|---|---|---|
| Ex 1 | 756,15 | 2,76 | 100 | 5 | OK | 3800 |
| Ex 2 | 755,5 | 2,76 | 100 | 0 | OK | 1900 |
| Ex 3 | - | 2,76 | 70 | 0 | OK | 1300 |
| Ex 4 | - | 2,76 | 50 | 0 | OK | 950 |
| Ex 5 | - | 2,76 | 30 | 0 | OK | 650 |
| Ex 6 | - | 2,76 | 100 | 19 | OK | 1200 |
| SEPIGEL® 305 | - | - | 0 | - | - | 620 |
| CEx 1 | - | 2,76 | 100 | 25 | OK | Précipitation |
| CEx 2 | - | 4 | 100 | 5 | Instable | NA |
| CEx 3 | - | 2,76 | 20 | 0 | OK | < 100 |
| CEx 4 | - | 1 | 100 | 0 | OK | < 100 |

Dans le Tableau 2, « masse phase aq » correspond à la masse de la phase aqueuse dans la composition, en grammes, et « [monomères] phase aq » correspond à la concentration de la totalité des monomères en solution aqueuse, en mmol par gramme de solution aqueuse.

Le Tableau 3 ci-dessous détaille les viscosités obtenues pour les exemples 1 et 2 ainsi que le SEPIGEL® 305, pour différents pourcentages massiques en polymère.

*Tableau 3 : mesure de la viscosité de compositions hydroalcooliques*

| Produit | %massique en polymère | Viscosité (cps) |
|---|---|---|
| SEPIGEL® 305 | 0,80% | 1950 |
| | 1,00% | 3500 |
| Exemple 1 | 0,20% | 510 |
| | 0,30% | 2100 |
| | 0,50% | 3800 |
| | 0,80% | 5800 |
| | 1,00% | 6000 |
| Exemple 2 | 0,30% | 100 |
| | 0,50% | 1900 |
| | 1,00% | 4500 |

Pour être qualifiée de gel, une composition doit avoir une viscosité supérieure à 500 cps.

Les polymères des exemples 1 à 6 donnent des compositions plus visqueuses que le SEPIGEL® 305 à concentration identique. Leur viscosité étant supérieure à 500 cps, ces compositions sont des gels.

S'agissant des contre-exemples, on remarque qu'un pourcentage d'acide faible neutralisé supérieur à 20% (CEx 1), tout autre paramètre restant inchangé, conduit à une précipitation du polymère, et ainsi à une composition hydroalcoolique non satisfaisante.

Une concentration de la totalité des monomères en solution aqueuse supérieure à 3,6 mmol par gramme de solution aqueuse (CEx 2) ou inférieure à 1,3 mmol par gramme de solution aqueuse (CEx 4), tout autre paramètre restant inchangé, conduit respectivement à une composition instable dont la viscosité ne peut être mesurée, ou à une composition dont la viscosité est plus faible que celle du SEPIGEL® 305, et notamment inférieure à 100 cps. Ces compositions ne sont pas des gels.

Un pourcentage molaire de monomères porteurs d'au moins une fonction acide faible par rapport à l'ensemble des monomères utilisés inférieur à 30 mol% (CEx 3), tout autre paramètre restant inchangé, conduit à une composition dont la viscosité est plus faible que celle du SEPIGEL® 305, et notamment inférieure à 100 cps. Cette composition n'est pas un gel.

Analyse sensorielle :

Une analyse sensorielle des compositions des exemples 1 à 6 (0,5% massique en polymère) et de la composition de SEPIGEL ® (1% massique en polymère) à iso-viscosité est réalisée avec la participation de 25 panelistes. Les panélistes évaluent les critères listés dans le Tableau 4 ci-dessous pour chacune de ces compositions hydroalcooliques, et leur attribuent une note de 0 à 5 pendant l'application et après l'application de ces compositions.

*Tableau 4 : critères d'analyse sensorielle*

| | Critères | 0 à 5 |
|---|---|---|
| Pendant l'application | Aspect freinant | Pas freinant à très freinant |
| | L'onctuosité | Pas onctueux à très onctueux |
| | La fraicheur | Pas frais à très frais |
| | L'aspect filant | Pas filant à très filant |
| | L'aspect collant | Pas collant à très collant |
| | Le temps de séchage | Très court à très long |
| Après application | L'aspect gras | Pas gras à très gras |
| | L'aspect collant | Pas collant à très collant |
| | La douceur | Pas doux à très doux |

Les résultats de l'analyse sensorielle sont illustrés sur la figure 1. Sur cette figure, les critères soulignés sont évalués après application de la composition, tandis que les critères non soulignés sont évalués pendant l'application de la composition.

Durant l'application, les polymères des exemples 1 à 6 de l'invention et le SEPIGEL® 305 présentent des caractéristiques similaires à savoir la sensation de fraicheur est jugée identique ainsi que l'onctuosité. En revanche, les polymères des exemples 1 à 6de l'invention se démarquent du SEPIGEL® 305 par une sensation de collant pendant l'application de la composition, jugée désagréable, beaucoup moins marquée mais surtout par un aspect filant pendant l'application de la composition totalement absent.

Après l'application de la composition, les panélistes ont jugé que les compositions des exemples 1 à 6 de l'invention laissaient une peau plus douce mais notent un temps de séchage plus longs (en moyenne de 6s), les autres paramètres étudiés restant identiques.

L'impression générale tend vers de meilleures qualités sensorielles pour les compositions préparées avec les exemples 1 à 6, par rapport à la composition préparée avec le SEPIGEL® 305.

**Revendications**

1. - Utilisation, pour la fabrication d'une composition hydroalcoolique comprenant 50% à 80% en masse d'au moins un alcool et au moins 10% en masse d'une phase aqueuse, d'un polymère branché ou réticulé composé de la répétition d'une ou plusieurs unités monomériques, dans lequel au moins une des unités monomériques comprend un groupe acrylique et au moins 30% molaire des unités monomériques sont porteuses d'au moins une fonction acide faible éventuellement sous forme neutralisée, ledit polymère étant obtenu :

par polymérisation d'une solution aqueuse d'un ou plusieurs monomères en émulsion inverse eau dans huile, dans laquelle au moins un des monomères utilisés est un monomère acrylique et au moins un des monomères utilisés est un monomère porteur d'au moins une fonction acide faible, le pourcentage molaire de monomères porteurs d'au moins une fonction acide faible par rapport à l'ensemble des monomères utilisés étant au moins de 30%, la phase aqueuse de la solution aqueuse contenant au moins un monomère jouant le rôle d'agent de ramification, de sorte que la polymérisation conduit à un polymère branché ou réticulé, et dans lequel :

i) la polymérisation est réalisée avec une concentration de la totalité des monomères en solution aqueuse appartenant à la gamme allant de 1,3 mmol à 3,6 mmol par gramme de solution aqueuse,
ii) pendant la polymérisation, au plus 20% molaire des fonctions acide présentes sur les monomères possédant au moins une fonction acide se trouvent sous forme neutralisée.

**2.** - Utilisation, pour épaissir une composition hydroalcoolique afin d'obtenir une composition hydroalcoolique épaissie comprenant 50% à 80% en masse d'au moins un alcool et au moins 10% en masse d'une phase aqueuse, d'un polymère branché ou réticulé composé de la répétition d'une ou plusieurs unités monomériques, dans lequel au moins une des unités monomériques comprend un groupe acrylique et au moins 30% molaire des unités monomériques sont porteuses d'au moins une fonction acide faible éventuellement sous forme neutralisée, ledit polymère étant obtenu :

par polymérisation d'une solution aqueuse d'un ou plusieurs monomères en émulsion inverse eau dans huile, dans laquelle au moins un des monomères utilisés est un monomère acrylique et au moins un des monomères utilisés est un monomère porteur d'au moins une fonction acide faible, le pourcentage molaire de monomères porteurs d'au moins une fonction acide faible par rapport à l'ensemble des monomères utilisés étant au moins de 30%, la phase aqueuse de le solution aqueuse contenant au moins un monomère jouant le rôle d'agent de ramification, de sorte que la polymérisation conduit à un polymère branché ou réticulé, et dans lequel :

i) la polymérisation est réalisée avec une concentration de la totalité des monomères en solution aqueuse appartenant à la gamme allant de 1,3 mmol à 3,6 mmol par gramme de solution aqueuse,
ii) pendant la polymérisation, au plus 20% molaire des fonctions acide présentes sur les monomères possédant au moins une fonction acide se trouvent sous forme neutralisée.

**3.** - Utilisation selon la revendication 1 ou 2, dans laquelle le polymère comprend un pourcentage de fonctions acide neutralisées de 30% à 100% molaire par rapport à la totalité des fonctions acide présentes sur le polymère, obtenu par une étape de neutralisation au moins partielle des fonctions acides présentes sur le polymère, réalisée après la polymérisation, et avant ou après la préparation de la composition.

**4.** - Utilisation selon l'une quelconque des revendications 1 à 3, dans laquelle la composition hydroalcoolique comprend :

a) 50 % à 80 % en masse dudit au moins un alcool,
b) 0,1 % à 10 % en masse du polymère branché ou réticulé,
c) 10 % à 49,9 % en masse d'eau,
d) 0 % à 10 % en masse d'au moins un additif.

**5.** - Utilisation selon l'une quelconque des revendications 1 à 4, dans laquelle la ou les unité(s) monomérique(s) porteuse(s) d'au moins une fonction acide faible, sous forme libre, est(sont) choisie(s) parmi l'acide acrylique, l'acide méthacrylique, l'acide itaconique, l'acide crotonique, l'acide maléique et l'acide fumarique, l'acide acrylique étant préféré.

**6.** - Utilisation selon l'une quelconque des revendications 1 à 5, dans laquelle le polymère est un copolymère comportant au moins une unité monomérique neutre choisie parmi l'acrylamide, le méthacrylamide, le N,N-diméthylacrylamide, le N-vinylméthylacétamide, le N-vinylformamide, l'acétate de vinyle, le diacétoneacrylamide, le N-isopropyl acrylamide, le N-[2-hydroxy-1,1-bis(hydroxyméthyl) éthyl] propénamide, l'acrylate de (2-hydroxyéthyle), l'acrylate de (2,3-dihydroxypropyle), le méthacrylate de méthyle, le méthacrylate de (2-hydroxyéthyle), le méthacrylate de (2,3-dihydroxy propyle) et la vinylpyrrolidone.

**7.** - Utilisation selon l'une quelconque des revendications 1 à 6, dans laquelle l'agent de ramification est choisi parmi le méthylènebisacrylamide (MBA), l'éthylène glycol di-acrylate, le polyéthylène glycol diméthacrylate, le diacrylamide, le cyanométhylacrylate, le vinyloxyéthylacrylate, le vinyloxyméthacrylate, la triallylamine, le formaldéhyde, le glyoxal, les glycidyléthers comme l'éthylèneglycol diglycidyléther, les époxy et leur mélange, la quantité d'agent de ramification étant comprise entre 5 ppm et 10 000 ppm en masse par rapport à la masse totale de monomères, et préférentiellement entre 100 ppm et 5 000 ppm.

**8.** - Composition hydroalcoolique, comprenant 50% à 80% en masse d'au moins un alcool, au moins 10% en masse d'une phase aqueuse, et un polymère branché ou réticulé composé de la répétition d'une ou plusieurs unités monomériques, dans lequel au moins une des unités monomériques comprenant un groupe acrylique et au moins 30% molaire des unités monomériques sont porteuses d'au moins une fonction acide faible au moins partiellement sous forme neutralisée, ledit polymère étant obtenu :
par polymérisation d'une solution aqueuse d'un ou plusieurs monomères en émulsion inverse eau dans huile, dans laquelle au moins un des monomères utilisés est un monomère acrylique et au moins un des monomères utilisés est un monomère porteur d'au moins une fonction acide faible, le pourcentage molaire de monomères porteurs d'au moins une fonction acide faible par rapport à l'ensemble des monomères utilisés étant au moins de 30%, la phase aqueuse de la solution aqueuse contenant au moins un monomère jouant le rôle d'agent de ramification, de sorte que la polymérisation conduit à un polymère branché ou réticulé, et dans lequel :

i) la polymérisation est réalisée avec une concentration de la totalité des monomères en solution aqueuse appartenant à la gamme allant de 1,3 mmol à 3,6 mmol par gramme de solution aqueuse,
ii) pendant la polymérisation, au plus 20% molaire des fonctions acide présentes sur les monomères possédant au moins une fonction acide se trouvent sous forme neutralisée.

**9.** - Composition hydroalcoolique selon la revendication 8, dans laquelle la polymérisation est suivie d'une étape de neutralisation au moins partielle des fonctions acides présentes sur le polymère, réalisée avant ou après l'incorporation du polymère dans la composition.

**10.** - Composition hydroalcoolique selon la revendication 8 ou la revendication 9, dans laquelle le polymère comprend un pourcentage de fonctions acide neutralisées de 30% à 100% molaire par rapport à la totalité des fonctions acide présentes sur le polymère, obtenu par l'étape de neutralisation.

**11.** - Composition hydroalcoolique selon l'une quelconque des revendications 8 à 10, ladite composition comprenant :

a) 50 % à 80 % en masse dudit au moins un alcool,
b) 0,1 % à 10 % en masse du polymère branché ou réticulé,
c) 10 % à 49,9 % en masse d'eau,
d) 0 % à 10 % en masse d'au moins un additif.

**12.** - Composition selon l'une quelconque des revendications 8 à 11, dans laquelle la ou les unité(s) monomérique(s) porteuse(s) d'au moins une fonction acide faible, sous forme libre, est(sont) choisie(s) parmi l'acide acrylique, l'acide méthacrylique, l'acide itaconique, l'acide crotonique, l'acide maléique et l'acide fumarique, l'acide acrylique étant préféré.

**13.** - Composition selon l'une quelconque des revendications 8 à 12, dans laquelle le polymère est un copolymère comportant au moins une unité monomérique neutre choisie parmi l'acrylamide, le méthacrylamide, le N,N-diméthylacrylamide, le N-vinylméthylacétamide, le N-vinylformamide, l'acétate de vinyle, le diacétoneacrylamide, le N-isopropyl acrylamide, le N-[2-hydroxy-1,1-bis(hydroxyméthyl) éthyl] propénamide, l'acrylate de (2-hydroxyéthyle), l'acrylate de (2,3-dihydroxypropyle), le méthacrylate de méthyle, le méthacrylate de (2-hydroxyéthyle), le méthacrylate de (2,3-dihydroxy propyle) et la vinylpyrrolidone.

**14.** - Composition selon l'une quelconque des revendications 8 à 13, dans laquelle l'agent de ramification est choisi parmi le méthylènebisacrylamide (MBA), l'éthylène glycol di-acrylate, le polyéthylène glycol diméthacrylate, le diacrylamide, le cyanométhylacrylate, le vinyloxyéthylacrylate, le vinyloxyméthacrylate, la triallylamine, le formaldéhyde, le glyoxal, les glycidyléthers comme l'éthylèneglycol diglycidyléther, les époxy et leur mélange.

**15.** - Composition selon l'une quelconque des revendications 8 à 14, dans laquelle la quantité d'agent de ramification est comprise entre 5 ppm et 10 000 ppm en masse par rapport à la masse totale de monomères, et préférentiellement entre 100 ppm et 5 000 ppm.

**Patentansprüche**

1.  - Einsatz zur Herstellung einer hydroalkoholischen Zusammensetzung mit 50 bis 80 Masse-% mindestens eines Alkohols und mindestens 10 Masse-% einer wässrigen Phase eines verzweigten oder vernetzten Polymers, zusammengesetzt aus der Wiederholung einer oder mehrerer Monomereinheiten, bei der mindestens eine der Monomereinheiten eine Acrylgruppe enthält und mindestens 30 Mol-% der Monomereinheiten mindestens eine schwache Säurefunktion tragen, eventuell in neutralisierter Form, wobei dieses Polymer erhalten wird durch:
    Polymerisierung einer wässrigen Lösung eines oder mehrerer Monomere in einer inverten Emulsion Wasser in Öl, bei der mindestens eins der verwendeten Monomere ein Acrylmonomer ist und mindestens eins der verwendeten Monomere Träger einer schwachen Säurefunktion ist, der Molprozentsatz der Monomere, die Träger mindestens einer schwachen Säurefunktion sind, beträgt, bezogen auf alle eingesetzten Monomere, mindestens 30% der wässrigen Lösung, die mindestens ein Monomer enthält, das die Rolle eines Vernetzungsmittels spielt, so dass die Polymerisierung, zu einem verzweigten oder vernetzten Polymer führt, und bei dem:

    i) die Polymerisierung mit einer Konzentration aller Monomere in wässriger Lösung innerhalb eines Bereichs zwischen 1,3 mmol und 3,6 mmol pro Gramm wässriger Lösung erfolgt,

    ii) während der Polymerisierung höchstens 20Mol-% der Säurefunktionen, die mindestens eine Säurefunktion besitzen, sich in einer neutralisierten Form befinden.

2.  Einsatz zur Verdickung einer hydroalkoholischen Zusammensetzung, um eine verdickte hydroalkoholische Zusammensetzung zu erhalten, die 50 bis 80 Masse-% mindestens eines Alkohols und mindestens 10 Masse-% einer wässrigen Phase eines verzweigten oder vernetzten Polymers, zusammengesetzt aus der Wiederholung einer oder mehrerer Monomereinheiten enthält, bei dem mindestens eine der Monomereinheiten eine Acrylgruppe umfasst und mindestens 30 Mol-% der Monomereinheiten mindestens eine schwache Säurefunktion tragen, eventuell in neutralisierter Form, wobei dieses Polymer erhalten wird durch:
    Polymerisierung einer wässrigen Lösung eines oder mehrerer Monomer in einer inverten Emulsion Wasser in Öl, bei der mindestens eins der verwendeten Monomere ein Acrylmonomer ist und mindestens eins der verwendeten Monomere Träger mindestens einer schwachen Säurefunktion ist, der Molprozentsatz an Monomeren, die Träger mindestens einer schwachen Säurefunktion sind, bezogen auf alle eingesetzten Monomere, beträgt mindestens 30% der wässrigen Lösung, die mindestens ein Monomer enthält, das die Rolle eines Verzweigungsmittels spielt, so dass die Polymerisierung, zu einem verzweigten oder vernetzten Polymer führt, und bei der:

    i) die Polymerisierung mit einer Konzentration erfolgt, bei der die Gesamtzahl der Monomere in wässriger Lösung innerhalb eines Bereichs zwischen 1,3 mmol und 3,6 mmol pro Gramm wässriger Lösung liegt.

    ii) während der Polymerisierung höchstens 20 Mol-% der Säurefunktionen, die sich auf den Monomeren befinden, die mindestens eine Säurefunktion besitzen, sich in einer neutralisierten Form befinden.

3.  Einsatz nach Anspruch 1 oder 2, bei dem das Polymer einen Prozentsatz neutralisierter Säurefunktionen zwischen 30 und 100 Mol-% bezogen auf die Gesamtheit der auf dem Polymer vorhandenen Säurefunktionen umfasst, erhalten durch einen Schritt der mindestens teilweisen Neutralisierung der auf dem Polymer vorhandenen Säurefunktionen, ausgeführt nach der Polymerisierung und vor oder nach der Herstellung der Zusammensetzung.

4.  Einsatz nach irgendeinem der vorstehenden Ansprüche 1 bis 3, bei dem die hydroalkoholische Zusammensetzung umfasst:

    a) 50 bis 80 Masse-% dieses mindestens einen Alkohols,
    b) 0,1 bis 10 Masse-% des verzweigten oder vernetzten Polymers,
    c) 10 bis 49,9 Masse-% Wasser,
    d) 0 bis 10 Masse-% mindestens eines Zusatzes.

5.  - Einsatz nach irgendeinem der vorstehenden Ansprüche 1 bis 4, bei dem die Monomereinheit(en), die mindestens eine schwache Säurefunktion, in freier Form trägt(tragen), ausgesucht wird(werden) unter Acrylsäure, Methacrylsäure, Itaconsäure, Crotonsäure, Maleinsäure und Fumarsäure, wobei Acrylsäure der Vorzug gegeben wird.

6.  Einsatz nach irgendeinem der vorstehenden Ansprüche 1 bis 5, bei dem das Polymer ein Copolymer ist, bestehend aus mindestens einer neutralen Monomereinheit, ausgewählt unter Acrylamid, Methacrylamid, N,N-dimethylacrylamid, N-vinylmethylacetamid, N-vinylformamid, Vinylacetat, Diacetonacrylamid, N-isopropyl acrylamid, N-[2-hydroxy-l,l- bis(hydroxymethyl) ethyl] propenamid, (2-hydroxyethyl)- Acrylat, (2,3-dihydroxypropyle)- Acrylat, Methylmethacrylat, (2- hydroxyethyl)- Methacrylat, (2,3-dihydroxy propyl)-Methacrylat und Vinylpyrrolidon.

7. Einsatz nach irgendeinem der vorstehenden Ansprüche 1 bis 6, bei dem das Verzweigungsmittel ausgewählt wird aus Methylenbisacrylamid (MBA), Ethylenglycoldi-acrylat, Polyethylenglycoldimethacrylat, Diacrylamid, Cyanomethylacrylat, Vinyloxyethylacrylat, Vinyloxymethacrylat, Triallylamin, Formaldehyd, Glyoxal, die Glycidylether wie Ethylenglycol- diglycidylether, die Epoxy und ihre Mischungen,
die Menge an Verzweigungsmittel liegt dabei zwischen 5 ppm und 10 000 ppm in Masse bezogen auf die Gesamtmasse Monomere und vorzugsweise zwischen 100 ppm und 5 000 ppm.

8. Hydroalkoholische Zusammensetzung mit 50 bis 80 Masse-% mindestens eines Alkohols und mindestens 10 Masse-% einer wässrigen Phase eines verzweigten oder vernetzten Polymers, zusammengesetzt aus der Wiederholung einer oder mehrerer Monomereinheiten, bei dem mindestens eine der Monomereinheiten eine Acrylgruppe enthält und mindestens 30 Mol-% der Monomereinheiten mindestens eine schwache Säurefunktion tragen, eventuell in neutralisierter Form, wobei dieses Polymer erhalten wird durch:
Polymerisierung einer wässrigen Lösung eines oder mehrerer Monomere in einer inverten Emulsion Wasser in Öl, bei der mindestens eins der verwendeten Monomere ein Acrylmonomer ist und mindestens eins der verwendeten Monomere Träger einer schwachen Säurefunktion ist, der Molprozentsatz der Monomere, die Träger mindestens einer schwachen Säurefunktion sind, bezogen auf alle eingesetzten Monomere beträgt mindestens 30% der wässrigen Lösung, die mindestens ein Monomer enthält, das die Rolle eines Verzweigungsmittels spielt, so dass die Polymerisierung, zu einem verzweigten oder vernetzten Polymer führt, und bei der:

    i) die Polymerisierung mit einer Konzentration erfolgt, bei der die Gesamtzahl aller Monomere in wässriger Lösung innerhalb eines Bereichs zwischen 1,3 mmol und 3,6 mmol pro Gramm wässriger Lösung liegt.
    ii) während der Polymerisierung höchstens 20 Mol-% der Säurefunktionen, die sich auf den Monomeren befinden, die mindestens eine Säurefunktion besitzen, sich in einer neutralisierten Form befinden.

9. Hydroalkoholische Zusammensetzung nach Anspruch 8, bei der die Polymerisierung gefolgt wird von einem Schritt der zumindest teilweisen Neutralisierung der auf dem Polymer vorhandenen Säurefunktionen, ausgeführt vor oder nach der Eingliederung des Polymers in die Zusammensetzung

10. Hydroalkoholische Zusammensetzung nach Anspruch 8 oder Anspruch 9, bei der das Polymer einen Prozentsatz neutralisierter Säurefunktionen von 30 bis 100 Mol-%, bezogen auf die Gesamtzahl der auf dem Polymer vorhandenen, durch den Schritt der Neutralisierung erhaltenen Säurefunktionen umfasst,

11. - Hydroalkoholische Zusammensetzung nach irgendeinem der vorstehenden Ansprüche 8 bis 10, diese Zusammensetzung umfasst:

    a) 50 bis 80 Masse-% dieses mindestens einen Alkohols,
    b) 0,1 bis 10 Masse-% des verzweigten oder vernetzten Polymers,
    c) 10 bis 49,9 Masse-% Wasser,
    d) 0 bis 10 Masse-% mindestens eines Zusatzes.

12. - Zusammensetzung nach irgendeinem der vorstehenden Ansprüche 8 bis 11, bei dem die Monomereinheit(en), die mindestens eine schwache Säurefunktion, in freier Form trägt(tragen), ausgesucht wird(werden) unter Acrylsäure, Methacrylsäure, Itaconsäure, Crotonsäure, Maleinsäure und Fumarsäure, wobei Acrylsäure der Vorzug gegeben wird.

13. Zusammensetzung nach irgendeinem der vorstehenden Ansprüche 8 bis 12, bei dem das Polymer ein Copolymer ist, bestehend aus mindestens einer neutralen Monomereinheit, ausgewählt unter Acrylamid, Methacrylamid, N,N-dimethylacrylamid, N- vinylmethylacetamid, N-vinylformamid, Vinylacetat, Diacetonacrylamid, N-isopropylacrylamid, N-[2-hydroxy-l,l- bis(hydroxymethyl) ethyl] propenamid, (2-hydroxyethyl)- Acrylat, (2,3-dihydroxypropyle)-Acrylat, Methylmethacrylat, (2- hydroxyethyl)-Methacrylat, (2,3-dihydroxy propyl)-Methacrylat und Vinylpyrrolidon

14. Zusammensetzung nach irgendeinem der vorstehenden Ansprüche 8 bis 13, bei dem das Verzweigungsmittel ausgewählt wird aus Methylenbisacrylamid (MBA), Ethylenglycoldi-acrylat, Polyethylenglycoldimethacrylat, Diacrylamid, Cyanomethylacrylat, Vinyloxyethylacrylat, Vinyloxymethacrylat, Triallylamin, Formaldehyd, Glyoxal, die Glycidylether wie Ethylenglycol - diglycidylether, die Epoxy und ihre Mischungen

15. Zusammensetzung nach irgendeinem der vorstehenden Ansprüche 8 bis 14, bei der die Menge an Verzweigungsmittel zwischen 5 ppm und 10 000 ppm in Masse, bezogen auf die Gesamtmasse Monomere und vorzugsweise zwischen 100 ppm und 5 000 ppm liegt.

**Claims**

1. - Use, for the manufacture of a hydroalcoholic composition comprising 50% to 80% by weight of at least one alcohol and at least 10% by weight of an aqueous phase, of a branched or crosslinked polymer composed of the repetition of one or more monomeric units, wherein at least one of the monomeric units comprises an acrylic group, and at least 30 mol% of the monomeric units bear at least one weak acid function, possibly in neutralized form, said polymer being obtained:
   by polymerization of an aqueous solution of one or more monomers in a water-in-oil inverse emulsion, wherein at least one of the monomers used is an acrylic monomer, and at least one of the monomers used is a monomer bearing at least one weak acid function, the molar percentage of monomers bearing at least one weak acid function relative to all the monomers used being at least 30%, the aqueous phase of the aqueous solution containing at least one monomer acting as a branching agent, so that the polymerization leads to a branched or crosslinked polymer, and wherein:

   i) the polymerization is carried out with a concentration of all the monomers in aqueous solution belonging to the range from 1.3 mmol to 3.6 mmol per gram of aqueous solution,
   ii) during the polymerization, at most 20 mol% of the acid functions present on the monomers having at least one acid function are in neutralized form.

2. - The use of a branched polymer or crosslinked compound consisting of the repetition of one or more monomeric units to thicken a hydroalcoholic composition in order to obtain a thickened hydroalcoholic composition comprising 50% to 80% by weight of at least one alcohol and at least 10% by weight of an aqueous phase, wherein at least one of the monomeric units comprises an acrylic group and at least 30 mol% of the monomeric units bear at least one weak acid function, optionally in neutralized form, said polymer being obtained:
   by polymerization of an aqueous solution of one or more monomers in a water-in-oil inverse emulsion, wherein at least one of the monomers used is an acrylic monomer and at least one of the monomers used is a monomer bearing at least one weak acid function, the molar percentage of monomers bearing at least one weak acid function relative to all the monomers used being at least 30%, the aqueous phase of the aqueous solution containing at least one monomer acting as a branching agent, so that the polymerization leads to a branched or crosslinked polymer, and wherein:

   i) the polymerization is carried out with a concentration of all the monomers in aqueous solution belonging to the range from 1.3 mmol to 3.6 mmol per gram of aqueous solution,
   ii) during the polymerization, at most 20 mol% of the acid functions present on the monomers having at least one acid function are in neutralized form.

3. - The use according to claim 1 or 2, wherein the polymer comprises a percentage of neutralized acid functions of 30% to 100 mol% relative to all of the acid functions present on the polymer, obtained by an at least partial neutralization step of the acid functions present on the polymer, carried out after the polymerization, and before or after the preparation of the composition.

4. - The use according to any of claims 1 to 3, wherein the hydroalcoholic composition comprises:

   a) 50% to 80% by weight of said at least one alcohol,
   b) 0.1% to 10% by weight of the branched or crosslinked polymer,
   c) 10% to 49.9% by weight of water,
   d) 0% to 10% by weight of at least one additive.

5. - The use according to any of claims 1 to 4, wherein the monomeric unit(s) bearing at least one weak acid function, in free form, is (are) chosen from acrylic acid, methacrylic acid, itaconic acid, crotonic acid, maleic acid and fumaric acid, acrylic acid being preferred.

6. - The use according to any of claims 1 to 5, wherein the polymer is a copolymer comprising at least one neutral monomeric unit chosen from acrylamide, methacrylamide, N,N-dimethylacrylamide, N-vinylmethylacetamide, N-vinylformamide, vinyl acetate, diacetoneacrylamide, N-isopropyl acrylamide, N-[2-hydroxy-1,1-bis(hydroxymethyl)ethyl] propenamide, (2-hydroxyethyl) acrylate, (2,3-dihydroxypropyl) acrylate, methyl methacrylate, (2-hydroxyethyl) methacrylate, (2,3-dihydroxypropyl) methacrylate and vinylpyrrolidone.

7. - The use according to any of claims 1 to 6, wherein the branching agent is selected from methylenebisacrylamide (MBA), ethylene glycol di-acrylate, polyethylene glycol dimethacrylate, diacrylamide, cyanomethylacrylate, vinyloxyethylacrylate, vinyloxymethacrylate, triallylamine, formaldehyde, glyoxal, glycidyl ethers such as ethylene glycol diglycidyl ether, epoxies and mixtures thereof, the amount of branching agent being between 5 ppm and 10,000 ppm by weight relative to the total weight of the monomers, and preferably between 100 ppm and 5,000 ppm.

8. - A hydroalcoholic composition, comprising 50% to 80% by weight of at least one alcohol, at least 10% by weight of an aqueous phase, and a branched or crosslinked polymer composed of the repetition of one or more monomeric units, wherein at least one of the monomer units comprises an acrylic group and at least 30 mol% of the monomer units bear at least one weak acid function, at least partially in neutralized form, said polymer being obtained:
by polymerization of an aqueous solution of one or more monomers in a water-in-oil inverse emulsion, wherein at least one of the monomers used is an acrylic monomer and at least one of the monomers used is a monomer bearing at least one weak acid function, the molar percentage of monomers bearing at least one weak acid function relative to all the monomers used being at least 30%, the aqueous phase of the aqueous solution containing at least one monomer acting as a branching agent, so that the polymerization leads to a branched or crosslinked polymer, and wherein:

i) the polymerization is carried out with a concentration of all the monomers in aqueous solution belonging to the range from 1.3 mmol to 3.6 mmol per gram of aqueous solution,
ii) during the polymerization, at most 20 mol% of the acid functions present on the monomers having at least one acid function are in neutralized form.

9. - The hydroalcoholic composition according to claim 8, wherein the polymerization is followed by a step of at least partial neutralization of the acid functions present on the polymer, carried out before or after the incorporation of the polymer into the composition.

10. - The hydroalcoholic composition according to claim 8 or claim 9, wherein the polymer comprises a percentage of neutralized acid functions of 30% to 100 mol% relative to all the acid functions present on the polymer, obtained by the neutralization step.

11. - The hydroalcoholic composition according to any of claims 8 to 10, said composition comprising:

a) 50% to 80% by weight of said at least one alcohol,
b) 0.1% to 10% by weight of the branched or crosslinked polymer,
c) 10% to 49.9% by weight of water,
d) 0% to 10% by weight of at least one additive.

12. - The composition according to any of claims 8 to 11, wherein the monomeric unit(s) bearing at least one weak acid function, in free form, is (are) chosen from acrylic acid, methacrylic acid, itaconic acid, crotonic acid, maleic acid and fumaric acid, acrylic acid being preferred.

13. - The composition according to any of claims 8 to 12, wherein the polymer is a copolymer comprising at least one neutral monomeric unit chosen from acrylamide, methacrylamide, N,N-dimethylacrylamide, N-vinylmethylacetamide, N-vinylformamide, vinyl acetate, diacetoneacrylamide, N-isopropyl acrylamide, N-[2-hydroxy-1,1-bis(hydroxymethyl)ethyl] propenamide, (2-hydroxyethyl) acrylate, (2,3-dihydroxypropyl) acrylate, methyl methacrylate, (2-hydroxyethyl) methacrylate, (2,3-dihydroxypropyl) methacrylate and vinylpyrrolidone.

14. - The composition according to any of claims 8 to 13, wherein the branching agent is chosen from methylenebisacrylamide (MBA), ethylene glycol di-acrylate, polyethylene glycol dimethacrylate, diacrylamide, cyanomethylacrylate, vinyloxyethylacrylate, vinyloxymethacrylate, triallylamine, formaldehyde, glyoxal, glycidyl ethers such as ethylene glycol diglycidyl ether, epoxies and mixtures thereof.

15. - The composition according to any of claims 8 to 14, wherein the amount of branching agent is between 5 ppm and 10,000 ppm by weight relative to the total weight of the monomers, and preferably between 100 ppm and 5,000 ppm.

**FIG. 1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2810545
- FR 2873126
- US 3724547 A
- EP 1047716 A
- JP H09157130 B